# EUROPEAN PATENT APPLICATION

(11) **EP 0 598 450 A1**
(43) Date of publication of application: **25.05.1994**
(21) Application number: 93203167.7
(22) Date of filing: 12.11.1993
(51) Int. Cl.: A61L 27/00

(54) **Prosthetic implant and method of making same**

(30) Priority: 12.11.1992 US 975292
(71) Applicant: Bristol-Myers Squibb Company, New York, N.Y. 10154 (US)
(72) Inventor: Devanathan, Thirumalai, Warsaw, IN 46580 (US)
(74) Representative: Mays, Julie

(57) **Abstract**

This invention provides for a prosthetic implant having a porous surface layer 22 connected to the implant body 20 by the use of a thin layer of a thermoplastic polymer 24 with a melting point substantially lower than the metal components and which exhibits good adhesive bonding characteristics. In the preferred embodiment the thermoplastic polymer of choice is from the polyaryletherketone family (PAEK).

The use of the thin film polymer bonding eliminates the undesirable effects incurred during the sintering process in two ways. First, since the thin film polymer melts at a substantially lower temperature than required during a sintering process, the implant body is not subjected to high temperature. Second, the porous coating and the implant are secured together without sintering; therefore, notches are not formed at the point of contact between the porous surface and the implant.

The resultant implant with a porous layer attached therefore retains substantially if not all the original strength characteristics of the implant body. Therefore, designers of prosthetic implants are not limited in the amount or location of the porous surface layer on the implant. Further, eliminating the sintering or diffusion bonding process reduces the cost incurred by the manufacturer in the form of time and capital expenditures required to sustain the high temperature furnaces.

## Description

### Field of the Invention

This invention relates to a prosthetic implant having a porous surface layer connected to the implant body by a thin film of adhesive thermoplastic polymer.

### Background of the Invention

Prosthetic implants for the replacement of a portion of a patient's joints are well known. Likewise, it is well known to provide a porous surface layer on the implant to promote the ingrowth of bone and enhance fixation of the implant within the bone. The porous surface layer may take the form of a plurality of small beads or a wire mesh. Commonly, the porous surface layer is sintered or diffusion bonded to the implant. Sintering or diffusion bonding requires that the implant and porous surface layer be heated to a temperature sufficient to cause the porous surface layer and implant body to fuse or melt together at their point of mutual contact. Typically this temperature is around 1800 degrees Fahrenheit or 980 degrees Celsius. If the sintered or diffusion bonded junction were viewed in cross section, a small notch would be seen extending into the implant on each side of a contact point between the porous surface layer and the implant. These notches decrease the mechanical strength of the implant. To compensate for the effect of the notches and maintain the strength of the implant above the minimum guidelines established by the FDA of 18.9 ksi, the prosthetic implant manufacturer designs the implant to ensure that the resultant strength is well above the established minimum. This may require the porous surface layer be limited only to areas of large cross sections to meet the design strength criterion. This may limit the manufacturer in the ability to place porous surface layers on smaller sized implants having smaller cross sections.

Further, the process of sintering requires submission of the implant to high temperatures (approximately 1800 degrees Fahrenheit; 980 degrees Celsius) for a predetermined period of time. The exposure of the implant body to high temperatures affects the mechanical strength of the implant. Therefore, manufacturers must also compensate for the reduction in strength attributed to the high temperatures.

### Summary of the Invention

This invention provides for a prosthetic implant having a porous surface layer connected to the implant body by the use of a thin layer of a thermoplastic polymer with a melting point substantially lower than the metal components and which exhibits good adhesive bonding characteristics. In the preferred embodiment, the thermoplastic polymer of choice is from the polyaryletherketone family (PAEK). Specifically, a PAEK sold by BASF under the trade name Ultrapek® A3000. The PAEK polymers have been shown through experimentation to exhibit strong adhesive bonds with molybdenum alloy (Co-Cr-Mo) and titanium-vanadium-aluminum alloy (Ti-6A1-4V) commonly used for implant materials.

The use of the thin film polymer bonding eliminates the undesirable effects incurred during the sintering process in two ways. First, since the thin film polymer melts at a substantially lower temperature than required during a sintering process, the implant body is not subjected to high temperature. Second, the porous coating and the implant are secured together without sintering, therefore notches are not formed at the point of contact between the porous surface and the implant.

The resultant implant with a porous layer attached therefore retains substantially if not all the original fatigue strength characteristics of the implant body. Therefore, designers of prosthetic implants are not limited in the amount or location of the porous surface layer on the implant. Further, eliminating the sintering or diffusion bonding process reduces the cost incurred by the manufacturer in the form of time and capital expenditures required to sustain the high temperature furnaces and tooling for example.

### Brief Description of the Drawings

Fig. 1 is an enlarged cross sectional view illustrating notches formed in a base during sintering.

Fig. 2 is a perspective view of the implant of the invention in the form of a prosthetic hip stem implant with portions sectioned for illustrative purposes.

Fig. 3 is an enlarged view of the junction between the implant body, the thermoplastic polymer, and the porous surface layer taken from the circled area of Fig. 2 identified by number 3.

### DESCRIPTION OF THE PREFERRED EMBODIMENT

The preferred embodiment herein described is not intended to be exhaustive or to limit the invention to the precise form disclosed. Rather, it is chosen and described to best explain the invention so that others skilled in the art might utilize its teachings.

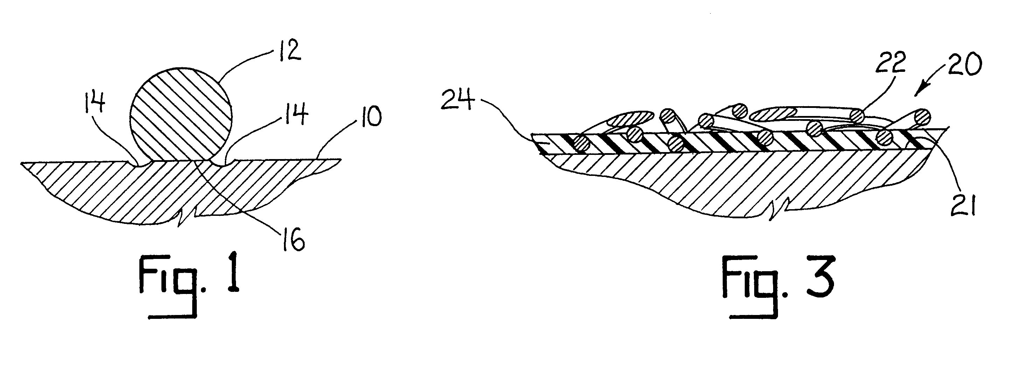

Referring now to Fig. 1, an enlarged cross section of the junction between the implant and porous layer affixed by sintering or diffusion bonding is illustrated. The base portion or body of the implant is represented by the numeral 10, the porous layer is represented by the numeral 12. It should be noted that only one bead or cross section or a single wire is illustrated. It should be understood that the terms sintering and diffusion bonding are used synonymously throughout this application as indicating a process using a high temperature to affect the metallurgical bond between two metals. Referring back to Fig. 1, a small notch 14 is created during the sintering process on both sides of the contact point 16 between the porous surface layer 12 and the implant body 10. Notches 14 extend into the implant body 10. If these notches 14 were viewed closer, it could be seen that the actual crystalline structure of the notch is altered relative to the remaining implant body. This alteration of the crystallin structure constitutes an inconsistency in the implant body which in turn reduces the fatigue strength of the implant. Further, if the outermost portion of the implant body were viewed relative to an innermost portion of the body, a change in the crystalline structure could be detected resulting in a further weakening. This reduction in strength has caused the implant designer to compensate for the reduction by limiting the area covered by the porous surface layer. Implants constructed in this manner have met with large success due to the care taken by the implant designers.

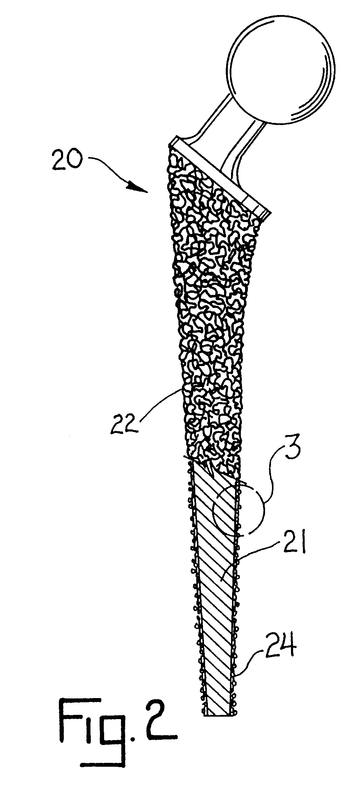

Referring now to Figs. 2 and 3, the invention is illustrated. The implant body 20 is illustrated in the form of prosthetic hip stem implant for insertion in the intramedullary canal of a prepared femur. A porous surface layer 22, in the form of wire mesh is connected to the implant body by a thin layer of thermoplastic polymer 24. In the preferred embodiment, the thermoplastic polymer of choice is from the polyaryletherketone family (PAEK). Specifically, a PAEK sold by BASF under the trade name Ultrapek® A3000. In the preferred embodiment when used to bond a fiber metal pad to the implant body, the thermoplastic layer is thicker than the diameter of the wire used to make the mesh. Typically, the thermoplastic layer will be approximately 20 thousandths of an inch thick ( approximately .05 millimeters). This provides for the encapsulation of at least a portion of the wires as illustrated in Fig. 3. If the thermoplastic polymer is used to bond a layer of beads to the implant, the layer may be much thinner as the adhesive bond will be relied upon to ensure fixation.

The implant of Figs. 2 and 3 is formed by placing a layer 24 of the thermoplastic polymer on either the implant body 21 or the porous surface layer 22. The two components with the thermoplastic polymer layer 24 positioned therebetween are then placed in contact with one another and subjected to an amount of pressure sufficient to hold the components in a tight relationship to one another. The components are then subjected to a heat of approximately 780 degrees fahrenheit (415 degrees Celsius). At this temperature, the thermoplastic polymer becomes molten. In its molten stage, the thermoplastic polymer is very sticky and readily adheres to the metal in the porous surface layer and the implant body. The thermoplastic polymer partially penetrates the porous surface layer. Once heated for a predetermined period of time, the components are allowed to cool to ambient temperature which returns the thermoplastic polymer to its solid state in a strong melt bond with the components. As illustrated in Fig. 3 a portion of the porous surface layer fibers are encapsulated in the layer 24 of thermoplastic polymer. The encapsulation enhances fixation by providing a mechanical interlock with the layer 24. The combination of the mechanical interlock and melt bond provides a strong bond between the porous surface layer and the implant.

Since the thermoplastic polymer melts at a low temperature, relative to the metal components, the crystalline structure of the metal implant is not adversely affected. Further, the melt bond between the thermoplastic polymer and the metal components does not create notches within the implant body. Therefore, the invention results in an implant having essentially if not all of the strength characteristics of the implant body. The designers using the invention would be able to construct any size of an implant with as much or as little porous surface layer as may be required for the application.

## Claims

1. A prosthetic implant comprising: a metal body, a porous surface layer and a thin layer of thermoplastic polymer positioned between the metal body and the porous surface layer to adhesively bond the porous surface layer to the metal body.

2. The prosthetic implant of Claim 1 wherein said thermoplastic polymer becomes molten at a temperature which is substantially less than the melting temperature of the metal body and porous surface layer.

3. The prosthetic implant of Claim 1 or Claim 2 wherein the thermoplastic polymer is from the polyaryletherketone family.

4. The prosthetic implant of any preceding claim wherein the thermoplastic polymer is a semicrystalline polymer.

5. A method of affixing a porous surface layer to a metal implant body without the formation of notches in the metal implant body and without subjecting the metal implant to temperatures in excess of 1,000 degrees fahrenheit (537 degrees celsius), said method comprising the steps of:
a) providing a prosthetic implant formed from a metal;
b) providing a porous surface layer;
c) providing a thin layer of a thermoplastic polymer;
d) forming an assembly of the porous surface layer in contact with the prosthetic implant with the thermoplastic polymer positioned therebetween;
e) heating the assembly to a temperature sufficient to transform the thin layer of thermoplastic polymer into a molten state to adhesively bind the metal implant body to the porous surface layer; and
f) cooling the assembly to ambient temperature to transform the thermoplastic polymer into a solid state:

6. The method of Claim 5 wherein the thermoplastic layer is from the polyaryletherketone family.

7. The method of Claim 5 or Claim 6 wherein the thermoplastic layer is a semicrystalline polymer.

8. The method of Claim 5, 6 or 7 wherein a portion of the porous surface layer is encapsulated by the layer of thermoplastic polymer.
